# EUROPÄISCHE PATENTSCHRIFT

(11) **EP 3 868 441 B1**
(45) Veröffentlichungstag und Bekanntmachung des Hinweises auf die Patenterteilung: **21.12.2022**
(21) Anmeldenummer: 21155601.4
(22) Anmeldetag: 05.02.2021
(51) Int. Cl.: A61N 5/10

(54) **STRAHLENTHERAPIEVORRICHTUNG, APPLIKATOR UND VERFAHREN ZUM GEWÄHRLEISTEN DER VERWENDUNG EINES ORDNUNGSGEMÄSSEN APPLIKATORS IN DER STRAHLENTHERAPIE**
RADIOTHERAPY DEVICE, APPLICATOR AND METHOD FOR ENSURING THE USE OF A PROPER APPLICATOR IN RADIOTHERAPY
DISPOSITIF DE RADIOTHÉRAPIE, APPLICATEUR ET PROCÉDÉ PERMETTANT DE GARANTIR L'UTILISATION D'UN APPLICATEUR APPROPRIÉ DANS LA RADIOTHÉRAPIE

(30) Priorität: 21.02.2020 DE 102020104628
(43) Veröffentlichungstag der Anmeldung: 25.08.2021
(73) Patentinhaber: Carl Zeiss Meditec AG, 07745 Jena (DE)
(72) Erfinder: SYMALLA, Michael, 73430 Aalen (DE); RÖDER, Norman, 99441 Döbritschen (DE)
(74) Vertreter: PATERIS Patentanwälte PartmbB

(56) Entgegenhaltungen:
- US-A1- 2014 008 443
- US-A1- 2016 339 267

## Beschreibung

Die vorliegende Erfindung betrifft einen Applikator zur intraoperativen Radiotherapie sowie eine Strahlentherapievorrichtung. Daneben betrifft die Erfindung ein Verfahren zum Gewährleisten der Verwendung eines ordnungsgemäßen Applikators in einer Strahlentherapievorrichtung.

Im Rahmen der intraoperativen Radiotherapie kurz IORT, werden zur Tumorbettbestrahlung sogenannte Applikatoren genutzt. Dabei erfolgt die Bestrahlung unter Aufsicht eines Chirurgen und eines Strahlentherapeuten, typischerweise während einer Operation in der geöffneten Körperhöhle. Dabei beeinflussen die verwendeten Applikatoren die Bestrahlung, da sie die im Rahmen der Radiotherapie applizierte Röntgenstrahlung absorbieren und den Abstand des zu bestrahlenden Gewebes von der Strahlenquelle beeinflussen. Der Einfluss der Applikatoren muss daher in der Bestrahlungsplanung berücksichtigt werden. Die für die Bestrahlungsplanung relevanten Daten des Applikators werden dabei auf einem Rechner gespeichert. Applikatoren für die in intraoperative Radiotherapie sowie Strahlentherapiesysteme, mit denen die Applikatoren Verwendung finden, sind beispielsweise in DE 10 2018 120 750 B3, DE 10 2008 030 590 A1, EP 2 335 778 A1 und WO 01/58346 A1 beschrieben.

Im Rahmen einer Radiotherapie wird typischerweise ein für die geöffnete Körperhöhle passender Applikator ausgewählt und in einem Menü der zum Erstellen des Bestrahlungsplanes verwendeten Software der ausgewählte Applikator ausgewählt. Die Erstellung des Bestrahlungsplans erfolgt dann auf der Basis des ausgewählten Applikators. Bei dieser Vorgehensweise ist jedoch nicht sichergestellt, dass der in der Software ausgewählte Applikator tatsächlich zur Anwendung kommt.

Zwar ist es beispielsweise aus dem Bereich der Implantate bekannt, Implantate mit einem RFID-Transponder zu versehen, auf dem Informationen zum Identifizieren des jeweiligen Implantates gespeichert sind, wobei die Informationen mittels eines RFID-Lesegerätes maschinell ausgelesen werden können. Aus US 2016/339267 A1 ist darüber hinaus ein Strahlentherapiesystem mit einem an einem Applikator angebrachten RFID-Transponder und einer Leseeinheit zum Auslesen des RFID-Transponders bekannt. Jedoch stellt auch die Verwendung eines solchen RFID-Transponders bei einem Applikator nicht sicher, dass tatsächlich derjenige Applikator zum Einsatz kommt, dessen Transpondersignal ausgelesen worden ist. So kann es beispielsweise, wenn mehrere Applikatoren zur Auswahl stehen, passieren, dass das Transpondersignal eines Applikators ausgelesen wird und dann versehentlich einer der anderen Applikatoren zur Anwendung kommt. Wenn sich sterile und nicht sterile Applikatoren in räumlicher Nähe zueinander befinden, kann ein solches Versehen schlimmstenfalls sogar dazu führen, dass ein Applikator Verwendung findet, dessen Sterilität nicht mehr gewährleistet ist. Dies kann bspw. passieren, wenn bei einem oder mehreren der zur Verfügung stehenden Applikatoren das Sterilitätsdatum abgelaufen ist und die Applikatoren bereits ausgepackt sind, so dass das auf der Verpackung aufgebrachte Sterilitätsdatum nicht mehr überprüft werden kann.

Es ist daher Aufgabe der vorliegenden Erfindung, einen vorteilhaften Applikator sowie eine vorteilhafte Strahlentherapievorrichtung zur Verfügung zu stellen, mit dem bzw. der die zuvor beschriebenen Problematiken zumindest teilweise überwunden werden können. Eine weitere Aufgabe der vorliegenden Erfindung ist es, ein Verfahren zum Verifizieren der Verwendung eines ordnungsgemäßen Applikators in einer Strahlentherapievorrichtung zur Verfügung zu stellen, mit dem die zuvor beschriebenen Problematiken zumindest teilweise überwunden werden können.

Diese Aufgaben werden durch einen Applikator nach Anspruch 1 bzw. durch eine Strahlentherapievorrichtung nach Anspruch 7 sowie durch ein Verfahren nach Anspruch 8 gelöst. Die abhängigen Ansprüche enthalten vorteilhafte Ausgestaltungen der Erfindung.

Ein erfindungsgemäßer Applikator zur intraoperativen Radiotherapie weist eine Aufnahme zum Aufnehmen einer Bestrahlungssonde auf, die insbesondere durch einen Hohlraum im inneren des Applikators gebildet sein kann. Außerdem weist er eine Einführöffnung zum Einführen der Bestrahlungssonde in die Aufnahme auf. Im erfindungsgemäßen Applikator ist die Einführöffnung mit einem Siegel verschlossen. Außerdem ist ein erster maschinell auslesbarer Informationsspeicher vorhanden, der zumindest teilweise derart an dem Siegel angeordnet ist, dass ein Entfernen oder Zerstören des Siegels die Auslesbarkeit des Informationsspeichers beseitigt. Der Informationsspeicher kann dabei eine Datamatrix, ein Barcode, ein Sender oder ein Transponder, insbesondere ein RFID-Transponder sein.

Mit Hilfe einer an der Strahlentherapievorrichtung, bei der der Applikator zum Einsatz kommen soll, vorhandenen Leseeinheit zum Auslesen des ersten Informationsspeichers kann mit Hilfe des ersten Informationsspeichers die Sterilität des Applikators verifiziert werden. Zum einen besteht die Möglichkeit, im ersten Informationsspeicher ein Sterilitätsdatum zu hinterlegen, nach dessen Ablauf die Sterilität nicht mehr gewährleistet ist und das automatisiert ausgelesen werden kann. Zum anderen führt die Tatsache, dass bei einem bereits ausgepackten Applikator das Siegel noch vorhanden ist und erst unmittelbar vor dem Einführen der Bestrahlungssonde entfernt zu werden braucht oder beim Einführen der Bestrahlungssonde zerstört wird, dazu, dass ein bereits benutzter Applikator an der mangelnden Auslesbarkeit des ersten Informationsspeichers automatisch erkannt werden kann. Es kann daher zuverlässig vermieden werden, dass ein bereits benutzter und daher nicht mehr steriler Applikator erneut benutzt wird. Der Informationsspeicher kann dabei insbesondere auch Informationen zum Identifizieren des Applikators enthalten, so dass auf der Basis der ausgelesenen Informationen auch der richtige Applikator in der Software zur Bestrahlungsplanung automatisiert eingestellt werden kann.

Um zu verhindern, dass zwischen dem Auslesen des Informationsspeichers und dem Fixieren des Applikators an der Strahlentherapievorrichtung eine Verwechslung des Applikators erfolgt, so dass ein anderer als im Bestrahlungsplan vorgesehener Applikator zur Anwendung kommt, ist es vorteilhaft, wenn der Applikator wenigstens einen zweiten maschinell auslesbaren Informationsspeicher umfasst, der wie der erste Informationsspeicher als Datamatrix, Barcode, Sender, Transponder, insbesondere als RFID-Transponder, etc. ausgestaltet sein kann. Vorteilhafterweise ist der zweite Informationsspeicher von derselben Art wie der erste Informationsspeicher. Der zweite Informationsspeicher enthält Identifikationsdaten des Applikators und ist entfernt vom Siegel angeordnet. Dadurch, dass der zweite Informationsspeicher vom Siegel entfernt angeordnet ist, kann verhindert werden, dass ein Entfernen oder Zerstören des Siegels die Auslesbarkeit des zweiten Informationsspeichers beeinträchtigt, so dass die durchgehende Auslesbarkeit der Identifikationsdaten gewährleistet ist. So kann auch noch bei einem fixierten Applikator verifiziert werden, dass es sich bei dem Applikator um dasselbe Modell handelt, dass auch der Bestrahlungsplan zugrunde liegt.

Der erste Informationsspeicher kann insbesondere ein erster RFID-Transponder sein. Entsprechend kann der zweite Informationsspeicher ein zweiter RFID-Transponder sein. RFID-Transponder sind besonders gut als Informationsspeicher geeignet, da sie nicht auf eine Energiequelle angewiesen sind und zudem bei Auslesen keine Sichtverbindung zur Leseeinheit zu bestehen braucht. Ein RFID-Transponder umfasst typischerweise einen Chip, in dem Daten gespeichert sind, und eine Antenne zum Senden der Daten und zum Empfangen der für das Senden benötigten Energie. Wenn der erste Informationsspeicher ein erster RFID-Transponder ist, umfasst der am Siegel angeordnete Teil des RFID-Transponders wenigstens die Antenne. Der RFID-Transponder kann aber auch vollständig am Siegel angeordnet sein.

Im erfindungsgemäßen Applikator kann gewährleistet werden, dass der erste Informationsspeicher nach Entfernen oder Zerstören des Siegels nicht mehr auslesbar ist, indem der am Siegel angeordnete Teil des ersten Informationsspeichers mit einer konstruktiven Schwachstelle, d.h. einer Sollbruchstelle versehen ist, an der er beim Entfernen oder Zerstören des Siegels zerstört wird. Wenn beispielsweise die Antenne eines RFID-Transponders beim Entfernen oder Zerstören des Siegels bricht, kann diese ihre Funktion nicht mehr erfüllen, so dass die im Chip gespeicherten Daten nicht mehr ausgelesen werden können.

Zusätzlich oder alternativ zur Sollbruchstelle kann der am Siegel angeordnete Teil des ersten Informationsspeichers derart am Siegel angeordnet sein, dass ein Durchstechen des Siegels zum Einführen der Bestrahlungseinrichtung in die Aufnahme des Applikators ohne Zerstörung des am Siegel angeordneten Teils des ersten Informationsspeichers nicht möglich ist. Falls der erste Informationsspeicher von einem RFID-Transponder gebildet wird, dessen Antenne am Siegel angeordnet ist, besteht die Möglichkeit, die Abstände zwischen den Abschnitte der Antenne und die Abmessungen der Leiterbahnen der Antenne im Hinblick auf die Abmessungen der Bestrahlungssonde derart zu wählen, dass die Querschnittsabmessung der Bestrahlungssonde größer als die Summe aus dem Abstand zwischen zwei Antennenabschnitten und der Breite der beiden Antennenabschnitte ist, so dass beim Durchstechen des Siegels mit der Bestrahlungssonde zumindest einer von zwei benachbarten Antennenabschnitten unterbrochen wird.

Erfindungsgemäß wird außerdem ein System mit einer Strahlentherapievorrichtung und einem erfindungsgemäßen Applikator zur Verfügung gestellt. Die Strahlentherapievorrichtung umfasst ein Gehäuse sowie eine vom Gehäuse ausgehende Bestrahlungssonde zum Einführen in den Applikator. Darüber hinaus umfasst die erfindungsgemäße Strahlentherapievorrichtung wenigstens eine Leseeinheit zum maschinellen Auslesen wenigstens eines maschinell auslesbaren Informationsspeichers des Applikators. Mit Hilfe der Leseeinheit können die Identifikationsdaten des zweiten Informationsspeichers ausgelesen werden, die dann mit den Identifikationsdaten des Applikators, der dem Bestrahlungsplan zugrunde gelegt ist, vergleichen werden können. Die Leseeinheit kann insbesondere eine Leseeinheit zum Auslesen eines RFID-Transponders sein. Aber auch eine Empfangseinheit zum Empfangen eines von einem Sender ausgesandten Signals oder eine optische Leseeinheit zum Auslesen eines Barcodes oder einer Datamatrix kommen, abhängig von der Art des am Applikator bzw. am Siegel vorhandenen Informationsspeichers, als Leseeinheit in Betracht.

Im erfindungsgemäßen Verfahren zum Verifizieren der Verwendung eines ordnungsgemäßen Applikators in einer Strahlentherapievorrichtung findet ein erfindungsgemäßer Applikator Verwendung. Beim Fixieren des Applikators an der Strahlentherapievorrichtung wird die maschinelle Auslesbarkeit des ersten Informationsspeichers überprüft, und es wird ein Warnsignal ausgegeben, wenn der ersten Informationsspeicher nicht auslesbar ist oder der erste Informationsspeicher auslesbar ist und ein abgelaufenes Sterilitätsdatum enthält. Nur solange das Siegel nicht entfernt oder zerstört ist, kann der erste Informationsspeicher ausgelesen werden, so dass die Auslesbarkeit des ersten Informationsspeichers ein unversehrtes Siegel impliziert, was wiederum auf die Sterilität des Applikators schließen lässt und ggf. das Feststellen des Sterilitätsdatums ermöglicht. Anhand des Warnsignals kann ein Nutzer des Applikators bei dessen Fixierung an der Strahlentherapievorrichtung automatisiert auf ein Problem mit der Sterilität des Applikators hingewiesen werden.

Falls der Applikator mit einem ersten und einem zweiten, Identifikationsdaten enthaltenden Informationsspeicher ausgestattet ist, kann das Verfahren im Rahmen einer Weiterbildung außerdem die folgenden Schritte umfassen:
- Erstes Auslesen der Identifikationsdaten aus dem zweiten Informationsspeicher des Applikators.
- Weitergeben der beim ersten Auslesen ausgelesenen Identifikationsdaten an die Strahlentherapievorrichtung.
- Zweites Auslesen der Identifikationsdaten aus dem zweiten Informationsspeicher des Applikators beim Fixieren des Applikators an der Strahlentherapievorrichtung.
- Vergleichen der beim zweiten Auslesen des zweiten Informationsspeichers ausgelesenen Identifikationsdaten mit den beim ersten Auslesen des zweiten Informationsspeichers ausgelesenen Identifikationsdaten und Ausgeben eines Warnsignals, wenn die beim zweiten Auslesen des zweiten Informationsspeichers ausgelesenen Identifikationsdaten nicht mit den beim ersten Auslesen des zweiten Informationsspeichers ausgelesenen Identifikationsdaten übereinstimmen.

Auf der Basis der beim ersten Auslesen ausgelesenen Identifikationsdaten kann die Auswahl des dem Bestrahlungsplan zugrunde liegenden Applikators erfolgen. Beim Fixieren des Applikators an der Strahlentherapievorrichtung kann dann eine Überprüfung erfolgen, ob tatsächlich derjenige Applikator verwendet wird, der dem Bestrahlungsplan zugrunde gelegt worden ist. Die Weiterbildung des erfindungsgemäßen Verfahrens ermöglicht somit nicht nur das automatisierte Verifizieren der Sterilität des verwendeten Applikators, sondern auch das automatisierte Verifizieren, dass der an der Strahlentherapievorrichtung fixierte Applikator mit dem dem Bestrahlungsplan zugrundeliegenden Applikator übereinstimmt. Auf diese Weise kann zuverlässig verhindert werden, dass ein falscher oder nicht steriler Applikator an der Strahlentherapievorrichtung fixiert wird.

Weitere Merkmale, Eigenschaften und Vorteile der vorliegenden Erfindung ergeben sich aus der nachfolgenden Beschreibung von Ausführungsbeispielen unter Bezugnahme auf die beiliegenden Figuren.
- Figur 1: zeigt ein erstes Ausführungsbeispiel für einen Applikator zur intraoperativen Radiotherapie.
- Figur 2: zeigt eine Strahlentherapievorrichtung mit einem daran fixierten Applikator.
- Figur 3: zeigt eine Draufsicht auf ein Siegel zum Verschließen der Einführöffnung eines Applikators.
- Figur 4: zeigt ein Verfahren zum Verifizieren der Verwendung eines ordnungsgemäßen Applikators in einer Strahlentherapievorrichtung.
- Figur 5: zeigt ein zweites Beispiel für einen Applikator zur intraoperativen Radiotherapie.

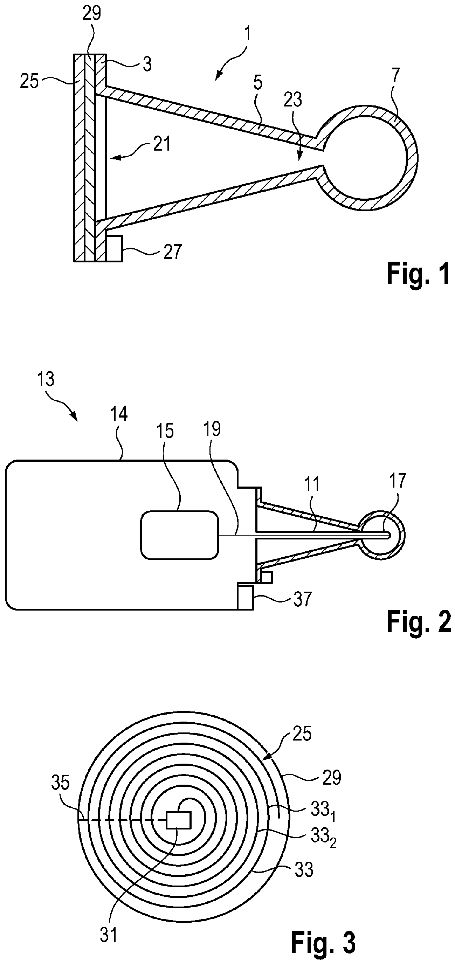

Ein erstes exemplarisches Ausführungsbeispiel für einen Applikator zur intraoperativen Radiotherapie ist in Figur 1 in einer Schnittansicht dargestellt. Der Applikator 1 des ersten exemplarischen Ausführungsbeispiels umfasst einen Flansch 3, von dem aus sich eine konisch zulaufende Hülse 5 erstreckt. An ihrem vom Flansch 3 entfernten Ende weist die Hülse 5 eine kugelförmige Ausbuchtung 7 auf. Der von der Hülse 5 und der kugelförmigen Ausbuchtung 7 umschlossene Hohlraum 23 bildet eine Aufnahme zum Aufnehmen einer Bestrahlungssonde 11, wie dies in Figur 2 dargestellt ist. Die Bestrahlungssonde 11 ist Teil einer Strahlentherapievorrichtung 13, die ein Gehäuse 14 mit einer darin angeordneten Elektronenstrahlquelle 15 aufweist. Die Bestrahlungssonde 11 erstreckt sich vom Gehäuse 14 aus bis zu einem distalen Ende 17 und bildet im vorliegenden exemplarischen Ausführungsbeispiel einen hohlen nadelförmigen Vorsprung der Strahlentherapievorrichtung. Ein von der Elektronenstrahlenquelle 15 ausgehender Elektronenstrahl 19 verläuft durch die hohle Bestrahlungssonde 11 bis zu deren distalen Ende 17, wo sie auf das Material des distalen Endes 17 auftrifft. Dabei werden die Elektronen des Elektronenstrahls 19 stark abgebremst, wobei Röntgenstrahlung entsteht, mit der das Tumorbett, welches bei Anwendung des Applikators typischerweise die Außenseite der kugelförmigen Ausbuchtung 7 des Applikators 1 kontaktiert, bestrahlt wird.

Zum Durchführen einer intraoperativen Radiotherapie wird die Bestrahlungssonde 11 durch eine Einführöffnung 21 im Bereich des Flansches 3 in den Hohlraum 23 eingeführt. Dadurch wird das distale Ende 17 der Bestrahlungssonde 11 im Bereich der kugelförmigen Ausbuchung 7 der Hülse 5 platziert. Die Außenfläche des Applikators 1, im vorliegenden Ausführungsbeispiel insbesondere die Außenfläche der kugelförmigen Ausbuchtung 7, wird während der intraoperativen Radiotherapie in eine Körperöffnung des Patienten eingeführt und mit dem Körpergewebe in Kontakt gebracht. Auf diese Weise lässt sich ein definierter Abstand des zu bestrahlenden Körpergewebes von der Quelle der Röntgenstrahlung, d.h. von dem distalen Ende 17 der Bestrahlungssonde 11 erreichen. Es sei an dieser Stelle darauf hingewiesen, dass der in Figur 1 dargestellte Applikator 1 lediglich ein exemplarisches Ausführungsbeispiel für eine mögliche Form eines Applikators 1 darstellt und dass eine Vielzahl möglicher Formen vorhanden sind, die u.a. von der Art zu bestrahlenden Gewebes und der geometrischen Konfiguration der Körperöffnung abhängen. Da der Applikator 1 während der intraoperativen Radiotherapie mit dem Gewebe in Kontakt kommt, muss er vor der Anwendung steril sein.

Für eine intraoperative Radiotherapie wird ein Bestrahlungsplan erstellt, der verschiedene Parameter des Applikators 1 berücksichtigen muss, etwa die Größe des Applikators 1, seine geometrische Form, das Material, aus dem er besteht, etc. Diese Parameter werden in eine Software eingegeben, mit deren Hilfe ein Bestrahlungsplan erstellt wird.

Der Applikator 1 des ersten exemplarischen Ausführungsbeispiels weist einen ersten RFID-Transponder 25 und einen zweiten RFID-Transponder 27 auf, die maschinell auslesbare Informationsspeicher darstellen. Der erste RFID-Transponder 25 enthält Daten zur Sterilität des Applikators 1, beispielsweise in Form eines Sterilitätsdatums, bis zu dem die Sterilität gewährleistet ist, und/oder eines Sterilisierungsdatums, an dem die Sterilisierung erfolgt ist. Der zweite RFID-Transponder 27 enthält Identifikationsdaten, mit deren Hilfe der Applikator 1 identifiziert werden kann. Die Identifikationsdaten umfassen eine eindeutige Kennung, beispielsweise in Form einer Identifikationsnummer, mit deren Hilfe der Applikator 1 eindeutig identifiziert werden kann. Darüber hinaus können die Identifikationsdaten auch Daten enthalten, die für die Bestrahlungsplanung relevant sind, etwa die radiologischen Daten des Applikators 1, den Typ des Applikators 1, seine Größe, seine geometrische Form, das Material, aus dem er besteht, etc. Auch eine Chargen- bzw. Seriennummer kann vorhanden sein.

Während der erste RFID-Transponder 25 auf einem Siegel 29 aufgebracht ist, mit dem die Einführöffnung 21 des Applikators 1 bis zu seiner Benutzung verschlossen ist, ist der zweite RFID-Transponder 27 abseits des Siegels 29 angebracht, im vorliegenden Ausführungsbeispiel an der vom Siegel 29 abgewandten Seite des Flansches 3.

Der auf dem Siegel angebrachte erste RFID-Transponder 25 weist einen die Daten enthaltenden Chip 31 und eine mit dem Chip 31 verbundene Antenne 33 auf (vgl. Figur 3). Über die Antenne 33 werden die im Chip 31 gespeicherten Daten an eine Leseeinheit 37 der Strahlentherapievorrichtung 13 gesendet. Außerdem empfängt der erste RFID-Transponder 25 die zum Senden benötigte Energie über die Antenne 33 von der Leseeinheit 37. Das Siegel 29 und die Antenne 33 weisen im vorliegenden Ausführungsbeispiel eine Sollbruchlinie 35 auf, entlang derer das Siegel 29 und die Antenne 33 reißen, wenn das Siegel 29 von der Einführöffnung 21 entfernt wird. Ein Auslesen des ersten RFID-Transponders 25 ist dann nicht mehr möglich, selbst dann, wenn sich das entfernte Siegel 29 in der Nähe der RFID-Leseeinheit 37 befindet. Falls das Siegel 29 so ausgebildet ist, dass vor dem Benutzen des Applikators 1 nicht von der Einführöffnung entfernt zu werden braucht, sondern beim Aufsetzen des Applikators 1 auf die Strahlentherapievorrichtung 13 von der Bestrahlungssonde 11 durchstochen wird, ist der erste RFID-Transponder 25 derart auf dem Siegel 29 derart auf dem Siegel 29 angeordnet, dass das Siegel 29 nicht durchstochen werden kann, ohne den ersten RFID-Transponder 25 derart zu beschädigen, dass dieser nicht mehr auslesbar ist. Dies kann beispielsweise dadurch geschehen, dass die Abstände zwischen benachbarten Antennenabschnitten 33₁, 33₂ so klein sind, dass das Siegel 29 nicht von der Bestrahlungssonde 11 durchstochen werden kann, ohne einen oder mehrere der Antennenabschnitte 33₁, 33₂ zu durchrennen. Ein gebrauchter Applikator 1 unterscheidet sich von einem nicht gebrauchten Applikator somit dadurch, dass der nicht gebrauchte Applikator 1 ein intaktes Siegel 29 mit einem auslesbaren ersten RFID-Transponder 25 aufweist. Bei einem gebrauchten, d.h. nicht mehr sterilen Applikator ist der erste RFID-Transponder 25 aufgrund der Zerstörung der Antenne 33 dagegen nicht mehr auslesbar.

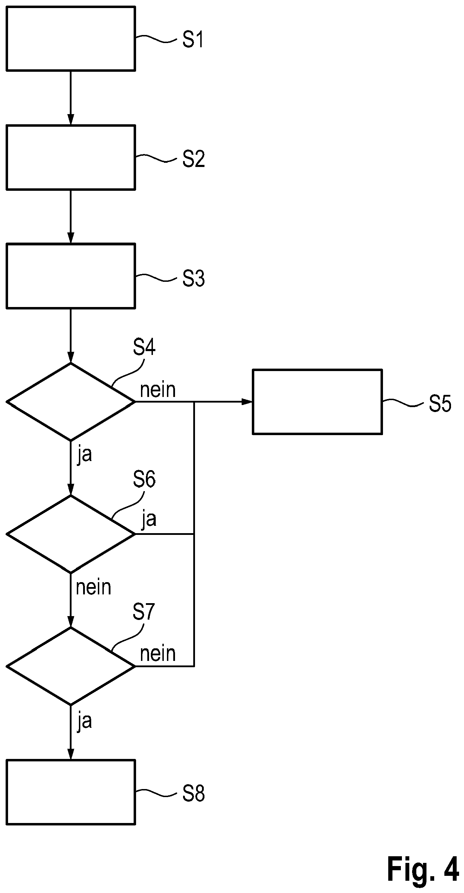

Mit Hilfe des ersten RFID-Transponders 25 und des zweiten RFID-Transponders 27 kann ein Verfahren zum Verifizieren der Verwendung eines ordnungsgemäßen Applikators 1 an einer Strahlentherapievorrichtung 13 durchgeführt werden. Dieses Verfahren ist schematisch in Figur 4 anhand eines Flussdiagramms dargestellt.

Zum Beginn des Verfahrens wird in Schritt S1 zumindest der zweite RFID-Transponder 27 von einem RFID-Lesegerät ausgelesen. Die im zweiten RFID-Transponder 27 gespeicherten Daten des Applikators werden dann in Schritt S2 an eine Einheit, mit deren Hilfe ein Bestrahlungsplan erstellt werden kann und die bspw. ein Computer sein kann, weitergegeben. Außerdem werden in Schritt S2 Identifikationsdaten, mit deren Hilfe sich der Applikator 1 eindeutig identifizieren lässt, etwa in Form einer Identifikationsnummer, von dem RFID-Lesegerät 37 an die Strahlentherapievorrichtung 13 weitergegeben. Das RFID-Lesegerät kann dabei die an der Strahlentherapievorrichtung angeordnete RFID-Leseeinheit 37 oder eine von dieser getrennte Leseeinheit, die bspw. in die Einheit, mit deren Hilfe ein Bestrahlungsplan erstellt werden kann, integriert ist, sein.

Nachdem ein Bestrahlungsplan erstellt worden ist, wird der Applikator 1 an der Strahlentherapievorrichtung 13 fixiert, wobei die an der Strahlentherapievorrichtung 13 angeordnete RFID-Leseeinheit 37 den ersten RFID-Transponder 25 in Schritt S3 ausliest. Außerdem wird im vorliegenden exemplarischen Ausführungsbeispiel der zweite RFID-Transponder 27 in Schritt S3 ein zweites Mal ausgelesen. Zum Auslesen der RFID-Transponder 25, 27 genügt es, wenn sich der Applikator 1 in Nähe der RFID-Leseeinheit 37 befindet, so dass das Auslesen des ersten RFID-Transponders 25 erfolgen kann, bevor das Siegel 29 von der Einführöffnung 21 des Applikators 1 entfernt wird oder von der Bestrahlungssonde 11 durchstochen wird.

In Schritt S4 wird dann überprüft, ob der erste RFID-Transponder 25 auslesbar ist. Ist dies nicht der Fall, so deutet dies auf eine Beschädigung des ersten RFID-Transponders 25 und somit auf eine nicht mehr vorhandene Sterilität des Applikators 1 hin. Die Strahlentherapievorrichtung 13 gibt dann ein optisches und/oder ein akustisches Warnsignal aus (Schritt S5). Wenn der erste RFID-Transponder 25 dagegen auslesbar ist, überprüft die Strahlentherapievorrichtung 13 in Schritt S6, ob das Sterilitätsdatum des Applikators 1 abgelaufen ist. Falls ja, schreitet das Verfahren zu Schritt S5 fort, in dem von der Strahlentherapievorrichtung 13 das Warnsignal ausgesandt wird. Falls das Verfallsdatum nicht überschritten ist, schreitet das Verfahren zu Schritt S7 fort.

Beim zweiten Auslesen des RFID-Transponders 27 (Schritt S3) werden zumindest die Identifikationsdaten, d.h. beispielsweise die Identifikationsnummer, des Applikators ausgelesen. In Schritt S7 vergleicht die Strahlentherapievorrichtung 13 dann die ausgelesenen Identifikationsdaten mit den beim ersten Auslesen des zweiten RFID-Transponders 27 ausgelesenen Identifikationsdaten. Falls dieser Vergleich ergibt, dass die Identifikationsdaten nicht miteinander übereinstimmen, schreitet das Verfahren zu Schritt S5 fort, in dem die Strahlentherapievorrichtung 13 ein Warnsignal ausgibt. Wenn andererseits in Schritt S7 eine Übereinstimmung der Identifikationsdaten festgestellt wird, wird das Verifizieren der Verwendung eines ordnungsgemäßen Applikators 1 in Schritt S8 beendet und die intraoperative Radiotherapie kann wie geplant durchgeführt werden.

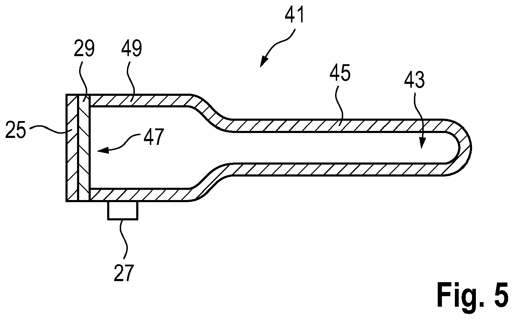

Ein alternatives exemplarisches Ausführungsbeispiel für einen erfindungsgemäßen Applikator ist in Figur 5 dargestellt. In diesem weist der Applikator 41 eine im Wesentlichen zylinderförmige Hülse 45 auf, deren Innenraum 43 eine Aufnahme für eine Bestrahlungssonde 11 bildet. Im Bereich einer Einführöffnung 47 ist der Applikator 41 des zweiten exemplarischen Ausführungsbeispiels wie der Applikator 1 des ersten exemplarischen Ausführungsbeispiels mit einem Siegel 29 verschlossen, auf dem der erste RFID-Transponder 25 angebracht ist. Für das Siegel 29 und den darauf aufgebrachten ersten RFID-Transponder 25 gilt das was mit Bezug auf das Siegel 29 und den ersten RFID-Transponder 25 des ersten Ausführungsbeispiels ausgeführt worden ist, entsprechend. Im Bereich der Eintrittsöffnung 47 weist die zylinderförmige Hülse 45 eine Aufweitung 49 auf, mit der der Applikator 41 auf einen Vorsprung einer Strahlentherapievorrichtung, vom den die Bestrahlungssonde ausgeht, aufgesetzt werden kann. Der zweite RFID-Transponder 27 ist im vorliegenden exemplarischen Ausführungsbeispiel an der Außenfläche des aufgeweiteten Abschnitts 49 der Hülse 45 angeordnet, so dass dieser beim Entfernen oder Durchstechen des Siegels 29 nicht beschädigt wird.

Die vorliegende Erfindung wurde anhand von exemplarischen Ausführungsbeispielen im Detail beschrieben. Ein Fachmann erkennt jedoch, dass Abweichungen von diesen Ausführungsbeispielen im Rahmen der vorliegenden Erfindung möglich sind. Insbesondere können Applikatoren mit andere geometrischen Formen Verwendung finden, wobei die Geometrien der distalen Enden der Applikatoren an die Geometrie der Körperöffnungen, in denen sie eingesetzt werden sollen, angepasst sind und die Hülsen der Applikatoren im Bereich ihrer Einführöffnungen an die Strahlentherapievorrichtungen, an denen sie fixiert werden sollen, angepasst sind. Darüber hinaus kann der zweite RFID-Transponder an anderen Stellen als den in den Ausführungsbeispielen gezeigten Stellen angeordnet sein, solange er bei einem Entfernen oder Durchstechen des Siegels nicht zerstört wird. Statt der RFID-Transponder können auch andere Datenspeicher Verwendung finden. Beispielsweise können Strichcodes oder Datamatrizen Verwendung finden, wobei RFID-Transponder jedoch den Vorteil haben, dass keine Sichtbeziehung zwischen der Leseeinheit und dem Datenspeicher vorhanden zu sein braucht. Zudem besteht die Möglichkeit, neben dem ersten RFID-Transponder und dem zweiten RFID-Transponder weitere Informationsspeicher, insbesondere weitere RFID-Transponder vorzusehen. Im Rahmen der beschriebenen Verfahrens zum Verifizieren der Verwendung eines ordnungsgemäßen Applikators kann auf das Auslesen des zweiten RFID-Transponders und das Vergleichen der Identifikationsdaten verzichtet werden, wenn lediglich die Verwendung eines sterilen Applikators verifiziert werden soll. Die vorliegende Erfindung soll daher nicht durch die exemplarischen Ausführungsbeispiele beschränkt sein, sondern lediglich durch die beigefügten Ansprüche.

### Bezugszeichenliste

- 1: Applikator
- 3: Flansch
- 5: Hülse
- 7: Ausbuchtung
- 11: Bestrahlungssonde
- 13: Strahlentherapievorrichtung
- 15: Elektronenstrahlquelle
- 17: distales Ende
- 19: Elektronenstrahl
- 21: Einführöffnung
- 23: Aufnahme
- 25: RFID-Transponder
- 27: RFID-Transponder
- 29: Siegel
- 31: Chip
- 33: Antenne
- 35: Sollbruchlinie
- 37: RFID-Leseeinheit
- 41: Applikator
- 43: Aufnahme
- 45: Hülse
- 47: Einführöffnung
- 49: aufgeweiteter Bereich
- S1: erstes Auslesen wenigstens des zweiten RFID-Transponders
- S2: Weitergabe der Identifikationsdaten an die Strahlentherapievorrichtung
- S3: Auslesen der RFID-Transponder
- S4: Überprüfung, ob der erste RFID-Transponder auslesbar ist
- S5: Ausgeben eines Warnsignals
- S6: Überprüfen, ob das im ersten RFID-Transponder gespeicherte Sterilitätsdatum abgelaufen ist
- S7: Überprüfen, ob die beim zweiten Auslesen des zweiten RFID-Transponders ausgelesenen Identifikationsdaten mit den beim ersten Auslesen des zweiten RFID-Transponders ausgelesenen Identifikationsdaten übereinstimmen
- S8: Beenden der Verifizierung

## Patentansprüche

1. Applikator (1, 41) zur intraoperativen Radiotherapie, der eine Aufnahme (23, 43) zum Aufnehmen einer Bestrahlungssonde (11) und eine Einführöffnung (21, 47) zum Einführen der Bestrahlungssonde (11) in die Aufnahme (23, 43) aufweist, **dadurch gekennzeichnet, dass** die Einführöffnung (21, 47) mit einem Siegel (29) verschlossen ist und dass ein erster maschinell auslesbarer Informationsspeicher (25) vorhanden ist, der zumindest teilweise derart an dem Siegel (29) angeordnet ist, dass ein Entfernen oder Zerstören des Siegels (29) die Auslesbarkeit des Informationsspeichers (25) beseitigt.

2. Applikator (1, 41) nach Anspruch 1, **dadurch gekennzeichnet, dass** er wenigstens einen zweiten maschinell auslesbareren Informationsspeicher (27) umfasst, der Identifikationsdaten des Applikators (1, 41) enthält und der entfernt vom Siegel (29) angeordnet ist.

3. Applikator (1, 41) nach Anspruch 1 oder Anspruch 2, **dadurch gekennzeichnet, dass** der erste Informationsspeicher ein erster RFID-Transponder (25) ist und/oder der zweite Informationsspeicher ein zweiter RFID-Transponder (27) ist.

4. Applikator (1, 41) nach Anspruch 3, **dadurch gekennzeichnet, dass** der erste RFID-Transponder (25) einen Chip (31) und eine Antenne (33) beinhaltet und der am Siegel (29) angeordneten Teil des RFID-Transponders (25) wenigstens die Antenne (33) umfasst.

5. Applikator (1, 41) nach einem der vorangehenden Ansprüche, **dadurch gekennzeichnet, dass** der am Siegel (29) angeordnete Teil des ersten Informationsspeichers (25) mit einer konstruierten Schwachstelle (35) versehen ist, an der er beim Entfern oder Zerstören des Siegels (29) zerstört wird.

6. Applikator (1, 41) nach einem der vorangehenden Ansprüche, **dadurch gekennzeichnet, dass** der am Siegel (29) angeordnete Teil des ersten Informationsspeichers (25) derart am Siegel (29) angeordnet ist, dass ein Durchstechen des Siegels (29) zum Einführen der Bestrahlungssonde (11) in die Aufnahme (23, 43) des Applikators (1, 41) ohne Zerstörung des am Siegel (29) angeordnete Teil des ersten Informationsspeichers (25) nicht möglich ist.

7. System mit einer Strahlentherapievorrichtung (13) und einem Applikator (1, 41) nach einem der vorangehenden Ansprüche, wobei die Strahlentherapievorrichtung (13) mit einem Gehäuse (14), einer vom Gehäuse (14) vorstehenden Bestrahlungssonde (11) zum Einführen in den Applikator (1, 41), und wenigstens einer Leseeinheit (37) zum maschinellen Auslesen wenigstens eines maschinell auslesbaren Informationsspeichers (25, 27) des Applikators (1, 41) ausgestattet ist.

8. Verfahren zum Verifizieren der Verwendung eines ordnungsgemäßen Applikators (1, 41) nach einem der Ansprüche 1 bis 6 in einer Strahlentherapievorrichtung (13), in dem beim Fixieren des Applikators (1, 41) an der Strahlentherapievorrichtung (13) die maschinelle Auslesbarkeit des ersten Informationsspeichers (25) überprüft wird und ein Warnsignal ausgegeben wird, wenn der erste Informationsspeicher (25) nicht auslesbar ist oder der erste Informationsspeicher (25) auslesbar ist und ein abgelaufenes Sterilitätsdatum enthält.

9. Verfahren nach Anspruch 8, welches außerdem die Schritte umfasst:
- erstes Auslesen der Identifikationsdaten aus dem zweiten Informationsspeicher (27) eines Applikators (1, 41) nach einem der Ansprüche 1 bis 6 und nach Anspruch 2;
- weitergeben der beim ersten Auslesen ausgelesenen Identifikationsdaten an die Strahlentherapievorrichtung (13);
- zweites Auslesen der Identifikationsdaten aus dem zweiten Informationsspeicher (27) des Applikators (1, 41) beim Fixieren des Applikators (1, 41) an der Strahlentherapievorrichtung (13);
- Vergleichen der beim zweiten Auslesen des zweiten Informationsspeichers (27) ausgelesenen Identifikationsdaten mit den beim ersten Auslesen des zweiten Informationsspeichers (27) ausgelesenen Identifikationsdaten und Ausgeben eines Warnsignals, wenn die beim zweiten Auslesen des zweiten Informationsspeichers (27) ausgelesenen Identifikationsdaten nicht mit den beim ersten Auslesen des zweiten Informationsspeichers (27) ausgelesenen Identifikationsdaten übereinstimmen.

## Claims

1. Applicator (1, 41) for intraoperative radiotherapy, which has a receptacle (23, 43) for receiving an irradiation probe (11) and an insertion opening (21, 47) for inserting the irradiation probe (11) into the receptacle (23, 43), **characterized in that** the insertion opening (21, 47) is closed with a seal (29), and **in that** there is a first machine-readable information store (25) which is at least partially arranged on the seal (29) in such a manner that removal or destruction of the seal (29) removes the ability to read the information store (25) .

2. Applicator (1, 41) according to Claim 1, **characterized in that** it comprises at least one second machine-readable information store (27) which contains identification data relating to the applicator (1, 41) and is arranged remotely from the seal (29).

3. Applicator (1, 41) according to Claim 1 or Claim 2, **characterized in that** the first information store is a first RFID transponder (25) and/or the second information store is a second RFID transponder (27).

4. Applicator (1, 41) according to Claim 3, **characterized in that** the first RFID transponder (25) comprises a chip (31) and an antenna (33), and that part of the RFID transponder (25) which is arranged on the seal (29) comprises at least the antenna (33).

5. Applicator (1, 41) according to one of the preceding claims, **characterized in that** that part of the first information store (25) which is arranged on the seal (29) is provided with a constructed weak point (35) at which it is destroyed when the seal (29) is removed or destroyed.

6. Applicator (1, 41) according to one of the preceding claims, **characterized in that** that part of the first information store (25) which is arranged on the seal (29) is arranged on the seal (29) in such a manner that it is not possible to pierce the seal (29), in order to insert the irradiation probe (11) into the receptacle (23, 43) of the applicator (1, 41), without destroying that part of the first information store (25) which is arranged on the seal (29).

7. System having a radiotherapy device (13) and an applicator (1, 41) according to one of the preceding claims, wherein the radiotherapy device (13) is equipped with a housing (14), an irradiation probe (11) which protrudes from the housing (14) and is intended to be inserted into the applicator (1, 41), and at least one reading unit (37) for machine-reading at least one machine-readable information store (25, 27) of the applicator (1, 41).

8. Method for verifying the use of a proper applicator (1, 41) according to one of Claims 1 to 6 in a radiotherapy device (13), in which the machine-readability of the first information store (25) is checked when fixing the applicator (1, 41) to the radiotherapy device (13) and a warning signal is output if the first information store (25) cannot be read or the first information store (25) can be read and contains an expired sterility date.

9. Method according to Claim 8, which also comprises the steps of:
- first reading of the identification data from the second information store (27) of an applicator (1, 41) according to one of Claims 1 to 6 and according to Claim 2;
- forwarding of the identification data read during the first reading to the radiotherapy device (13);
- second reading of the identification data from the second information store (27) of the applicator (1, 41) when fixing the applicator (1, 41) to the radiotherapy device (13);
- comparison of the identification data read during the second reading of the second information store (27) with the identification data read during the first reading of the second information store (27) and outputting of a warning signal if the identification data read during the second reading of the second information store (27) do not match the identification data read during the first reading of the second information store (27).

## Revendications

1. Applicateur (1, 41) destiné à la radiothérapie peropératoire, lequel applicateur comporte un logement (23, 43) destiné à recevoir une sonde de rayonnement (11) et une ouverture d'insertion (21, 47) destinée à insérer la sonde de rayonnement (11) dans le logement (23, 43), **caractérisé en ce que** l'ouverture d'insertion (21, 47) est fermée par un élément de scellement (29) et **en ce qu'**une première mémoire d'informations (25) lisible par machine est prévue qui est au moins partiellement disposée au niveau de l'élément de scellement (29) de manière à ce que le retrait ou la destruction de l'élément de scellement (29) supprime la possibilité de lire la mémoire d'informations (25).

2. Applicateur (1, 41) selon la revendication 1, **caractérisé en ce qu'**il comprend au moins une deuxième mémoire d'informations (27) lisible par machine qui contient des données d'identification de l'applicateur (1, 41) et qui est disposée à distance de l'élément de scellement (29).

3. Applicateur (1, 41) selon la revendication 1 ou la revendication 2, **caractérisé en ce que** la première mémoire d'informations est un premier transpondeur RFID (25) et/ou la deuxième mémoire d'informations est un deuxième transpondeur RFID (27).

4. Applicateur (1, 41) selon la revendication 3, **caractérisé en ce que** le premier transpondeur RFID (25) contient une puce (31) et une antenne (33) et la partie du transpondeur RFID (25), qui est disposée au niveau de l'élément de scellement (29), comprend au moins l'antenne (33).

5. Applicateur (1, 41) selon l'une des revendications précédentes, **caractérisé en ce que** la partie de la première mémoire d'informations (25), qui est disposée au niveau de l'élément de scellement (29), est munie d'un point de faiblesse construit (35) au niveau duquel ladite partie peut être détruite lorsque l'élément de scellement (29) est retiré ou détruit.

6. Applicateur (1, 41) selon l'une des revendications précédentes, **caractérisé en ce que** la partie de la première mémoire d'informations (25), qui est disposée au niveau de l'élément de scellement (29), est disposée au niveau de l'élément de scellement (29) de manière à ce que le perçage de l'élément de scellement (29) afin d'insérer la sonde de rayonnement (11) dans le logement (23, 43) de l'applicateur (1, 41) n'est pas possible sans détruire la partie de la première mémoire d'informations (25) qui est disposée au niveau de l'élément de scellement (29).

7. Système comprenant un dispositif de radiothérapie (13) et un applicateur (1, 41) selon l'une des revendications précédentes, le dispositif de radiothérapie (13) étant conçu avec un boîtier (14), une sonde de rayonnement (11) faisant saillie du boîtier (14) afin d'être insérée dans l'applicateur (1, 41), et au moins une unité de lecture (37) destinée à la lecture par machine d'au moins une mémoire d'informations (25, 27), lisible par machine, de l'applicateur (1, 41).

8. Procédé de vérification de l'utilisation d'un applicateur approprié (1, 41) selon l'une des revendications 1 à 6 dans un dispositif de radiothérapie (13), procédé dans lequel, lors de la fixation de l'applicateur (1, 41) au dispositif de radiothérapie (13), la possibilité de lire par machine la première mémoire d'informations (25) est vérifiée et un signal d'avertissement est délivré si la première mémoire d'informations (25) ne peut pas être lue ou si la première mémoire d'informations (25) peut être lue et contient une date de stérilité expirée.

9. Procédé selon la revendication 8, comprenant en outre les étapes suivantes :
- effectuer une première lecture des données d'identification de la deuxième mémoire d'informations (27) d'un applicateur (1, 41) selon l'une des revendications 1 à 6 et selon la revendication 2 ;
- transmettre les données d'identification, lues lors de la première lecture, au dispositif de radiothérapie (13) ;
- effectuer une deuxième lecture des données d'identification dans la deuxième mémoire d'informations (27) de l'applicateur (1, 41) lors de la fixation de l'applicateur (1, 41) au dispositif de radiothérapie (13) ;
- comparer les données d'identification, lues lors de la deuxième lecture de la deuxième mémoire d'informations (27), aux données d'identification lues lors de la première lecture de la deuxième mémoire d'informations (27) et délivrer un signal d'avertissement si les données d'identification lues lors de la deuxième lecture de la deuxième mémoire d'informations (27) ne correspondent pas aux données d'identification lues lors de la première lecture de la deuxième mémoire d'informations (27).
